(19) Europäisches Patentamt
European Patent Office
Office européen des brevets

(11) EP 2 281 805 A1

(12) EUROPEAN PATENT APPLICATION

(43) Date of publication:
**09.02.2011 Bulletin 2011/06**

(21) Application number: **09176526.3**

(22) Date of filing: **19.11.2009**

(51) Int Cl.:
*C07C 231/24* (2006.01) *C07C 237/46* (2006.01)
*C07B 63/00* (2006.01) *B01J 20/281* (2006.01)

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **21.07.2009 US 227103 P**

(71) Applicant: **GE Healthcare AS**
**0401 Oslo (NO)**

(72) Inventors:
- **Holmaas, Lars, Terje**
  **4521 Spangereid (NO)**
- **Homestad, Ole, Magne**
  **4521 Spangereid (NO)**
- **Malthe-Sørenssen, Didrik**
  **4521 Spangereid (NO)**
- **Pressman, Eric**
  **East Greenbush, NY 12061 (US)**
- **Stella, Albert**
  **Voorheesville, NY 12186 (US)**

(74) Representative: **Wulff, Marianne Weiby et al**
**GE Healthcare AS**
**P.O. Box 4220 Nydalen**
**Nycoveien 1-2**
**0401 Oslo (NO)**

# (54) An adsorptive purification method for iodixanol

(57) This invention relates to a method of using solid adsorbents to reduce backpeaks content in the purification of iodixanol. In particular, it relates to the use of non-polar organic adsorbents with the average pore diameter smaller than 30 nm. Specific examples of adsorbents of the instant invention include non-polar acrylic ester, di-vinyl benzene resins, poly-styrene di-vinyl benzene resins, and carbon adsorbents. In certain embodiments, upwards of 30% of the backpeak levels and 60% N-acetyl cyclic iodixanol levels are reduced for a 5% loss of iodixanol.

EP 2 281 805 A1

Printed by Jouve, 75001 PARIS (FR)

## Description

### TECHNICAL FIELD

This invention relates generally to non-ionic X-ray contrast agents. It further relates to a method of using solid adsorbents to reduce backpeaks content in the purification of iodixanol. In particular, it relates to the use of non-polar organic adsorbents with the average pore diameter smaller than about 30 nm.

### BACKGROUND OF THE INVENTION

Iodixanol is a non-ionic X-ray contrasting agent produced in large quantities by GE Healthcare in Lindesnes, Norway. The industrial production of iodixanol involves a multistep chemical synthesis. The lowest yield is in the final step, involving coupling of the intermediate 5-acetamido-N,N'-bis(2,3-dihydroxypropyl)-2,4,6-triiodoisophthalamide ("Compound A") with epichlorohydrin under basic conditions. Yield losses are attributed to side reactions leading to byproducts ("backpeaks"). These backpeaks must be minimized to provide iodixanol that meets regulatory requirements like those mandated by US Pharmacopeia.

While the purification of iodixanol is necessary and extensive, it is also important that such process does not lead to substantial loss of iodixanol. There exists a need for economic procedures to achieve the desired purity of iodixanol, while minimizing the loss of the final product during the purification process.

### BRIEF DESCRIPTION OF THE DRAWINGS

**FIG. 1** illustrates iodixanol purification in aqueous solution with various mostly non-polar adsorbents.

**FIG. 2** illustrates aqueous iodixanol solution backpeak reduction from control for best non-polar adsorbents.

**FIG. 3** illustrates aqueous iodixanol solution N-acetyl cyclic iodixanol reduction using mostly non-polar adsorbents.

**FIG. 4** illustrates backpeak adsorption isotherms from aqueous solution for CG71M resin and BGHHM and NC01-125 carbons.

**FIG. 5** illustrates iodixanol adsorption isotherms from aqueous solution for CG71M resin and BGHHM and NC01-125 carbons

**FIG. 6** illustrates average performance of adsorbents for purifying iodixanol in a 20.8 wt% aqueous solution, showing the percentage of the original mass adsorbed by the adsorbent.

**FIG. 7** illustrates average separation factors for iodixanol to backpeaks and iodixanol to N-acetyl cyclic iodixanol in aqueous solutions at 25°C

**FIG. 8** illustrates cumulative pore volume as a function of average pore size for BGHHM Carbon.

### SUMMARY OF THE INVENTION

The present invention provides a large scale purification process of iodixanol. This process involves the use of non-polar organic adsorbents in the treatment of bulk iodixanol, where the average pore diameter is smaller than about 30 nm. Specific examples of adsorbents of the instant invention include non-polar acrylic ester, di-vinyl benzene resins, poly-styrene di-vinyl benzene resins, and carbon adsorbents. In some preferred embodiments, adbsorbents with 90% of the pore volume smaller than 30 nm are employed. In other embodiments, adsorbents with surface area for adsorption about 1350 $m^2$/g of adsorbent are used.

The less polar impurities include N-acetyl cyclized iodixanol, cyclized iodixanol, and primary o-alkyl trimer. In certain embodiments, upwards of 30% of the backpeak levels and 60% N-acetyl cyclic iodixanol levels are reduced for a 5% loss of iodixanol. For example, upwards of 30% of the backpeak levels reductions may be obtained for a 2:1 solids to adsorbent mass ratio. In another example, separation factors of four may be obtained between iodixanol and backpeaks in adsorptive purification, with about a 20% reduction in backpeaks for a 5% loss of iodixanol to the adsorbed phase.

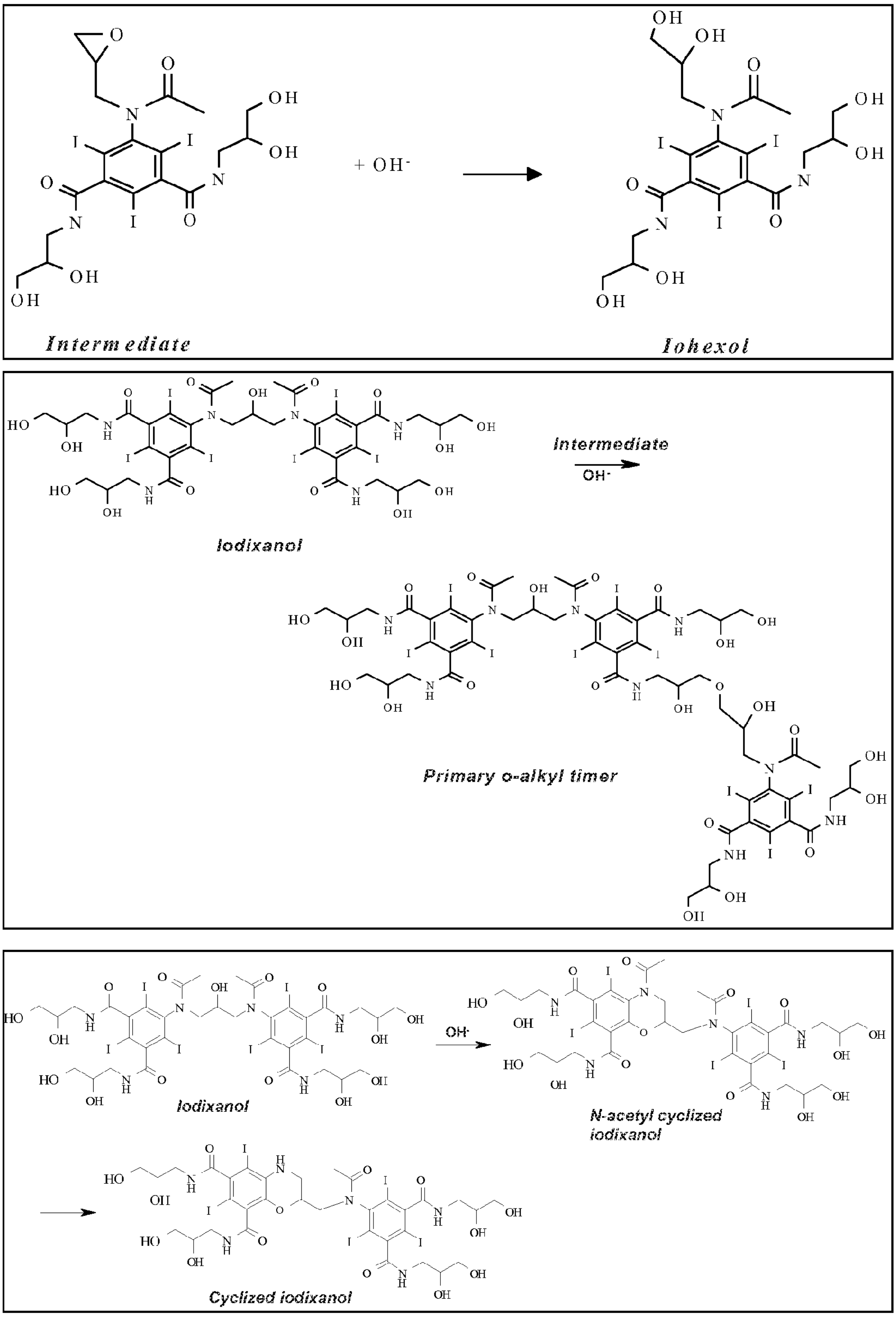
+ OH-
Intermediate
Iohexol
Intermediate
OH-
Iodixanol
Primary o-alkyl timer
OH-
Iodixanol
N-acetyl cyclized iodixanol
Cyclized iodixanol

Scheme 1

## DETAILED DESCRIPTION OF THE INVENTION

The instant invention is directed to purifying bulk iodixanol using non-polar organic adsorbents, where the average pore diameter is smaller than about 30 nm. In a preferred embodiment, analyses on iodixanol solutions before and after treatment with the adsorbents are performed by High Performance Liquid Chromatography (HPLC). For example, based on a HPLC assay, backpeaks as the major contaminants may be at 0.80-0.90 wt%.

In some embodiments, water is chosen as the solvent for iodixanol in order to exploit the more non-polar nature of the backpeaks relative to iodixanol. This is to create a very polar interaction of iodixanol with water, and a more hydrophobic environment on the adsorbent, to selectively remove backpeaks. This strategy is consistent with the results of reversed phase HPLC conditions, where backpeaks are selectively adsorbed to a greater extent (i.e. have longer retention times than iodixanol) onto a non-polar C18 column when eluting a primarily aqueous mobile phase.

Among the preferred adsorbents selected were the non-polar acrylic ester and di-vinyl benzene resins, as well as a set of poly-styrene di-vinyl benzene resins, and a number of carbon adsorbents. For contrast, a set of polar silica adsorbents was also studied. A 20.8 wt% solids aqueous iodixanol solution was contacted with the adsorbents at 25°C in a mass ratio of 2:1 iodixanol to adsorbent in order to see if there was a dramatic effect on iodixanol purity with the high loading of adsorbent (Figure 1).

The data show that certain types of carbon and acrylic ester resin adsorbents purify the iodixanol from the less polar backpeaks and N-acetyl cyclic iodixanol in aqueous solution. The amount of backpeak reduction is shown in Figure 2 for the three carbons and the acrylic ester resin that performed the best in purifying the iodixanol. These four adsorbents reduced the backpeak level by 30% or more and are all commercially available. In addition, cyclic iodixanol is also reduced by 65-70% as shown in Figure 3.

Although the CG71M has a lower surface area (500m$^2$/g) than BGHHM Carbon (1350 m$^2$/g), one important comparison for adsorbents is capacity for the desired material to adsorb relative to the product. The preferred way to test this is to perform isotherm studies. To measure adsorption isotherms, known weights of dry adsorbent were added to aliquots of the standard aqueous iodixanol solution of 20.8 wt% solids in mass ratios of 4:1 up to 30:1 iodixanol to adsorbent. The slurries were contacted with mixing for 24 hours, and then aliquots of the resulting supernatant liquid were analyzed for iodixanol, backpeak and N-acetyl cyclic iodixanol content by the standard HPLC analysis.

The results for backpeak adsorption as a function of concentration are shown in Figure 4. The data are plotted as amount of backpeak adsorbed per mass of adsorbent versus the mass concentration of backpeak in the solution at equilibrium.

The scatter in Figure 4 is due to several factors. The measurements are done by dilution of 200 micro-liters of the supernatant liquid with water and then injection into the HPLC. Although the masses are recorded to four decimal places, there are still potential inaccuracies associated with taking a small sample of the solution. In addition, to get a measured mass concentration from the HPLC, the area for the peak from the HPLC UV measurement at 254 nm is correlated for the control samples, knowing the concentration of each component in the control. The area response of the adsorbed samples is then taken as a ratio of it to the control in order to determine the mass concentration of the sample. In addition, there could be some imprecision involved in the manual integration of the peaks. Despite the scatter, the data show that BGHHM has a better capacity for backpeaks than NC01-125.

Similar results are shown for iodixanol adsorption as a function of loading. Figure 5 shows that all adsorbents adsorb only fairly small amounts of iodixanol relative to the solution concentration of nearly 20-wt%.

To examine the isotherm data, the average performance of each adsorbent may be plotted. Because the overall shape of the isotherms over these concentration ranges is fairly flat, the average will provide some relative performance of the adsorbent at purifying iodixanol. Such a plot is shown in Figure 6. The performance is based on the percentage of the original mass in the solution that is adsorbed by the adsorbent. The standard deviations are also given, and are large due to the nature of the average measurement being done over a fairly wide concentration range. On average, all three adsorbents remove about 15-25% of the initial backpeak level (low concentration), while adsorbing only 4-7% of the iodixanol (high concentration). This indicates that the adsorbents are highly selective for the backpeaks relative to the iodixanol.

From the measurements, an average separation factor can also be calculated. The separation factor is defined as the ratio of the mole fractions of components i and j in phases 1 and 2. *See* King, C.J. Separation Processes. 1980: McGraw-Hill.

$$\alpha_{ij} = \frac{x_{i1} / x_{j1}}{x_{i2} / x_{j2}} \qquad \text{Eq. 1}$$

For $\alpha ij>1$, component i will be concentrated in phase 1, and component j in phase 2. In this case, iodixanol is component i and backpeak or N-cyclized is component j, with phase 1 being the solution and phase 2 being the solid adsorbent. As can be seen in Figure 7, the average separation factors for iodixanol to backpeaks and iodixanol to N-acetyl cyclic iodixanol are shown. All are greater than one, indicating that iodixanol is concentrated in the solution phase, and the backpeaks and N-acetyl cyclic iodixanol are concentrated in the adsorbed phase. The larger the positive number, the better the separation. The N-acetyl cyclic iodixanol is less polar and thus separated better from the iodixanol than the backpeaks. All three non-polar adsorbents are about equal at backpeak separation, with the CG71M and the NC01-125 being better at N-acetyl cyclic iodixanol separation. This separation factor comparison may be a preferred method of comparing adsorption performance over the range of concentrations than the isotherms, due to the potential inaccuracies in calculations noted above.

A study of Calgon BGHHM carbon pore size distribution (see Figure 8) was performed, and is shown to give an example of the pore sizes that contribute to the adsorptive purification of iodixanol. In this way, the preferred pore sizes for purification may be determined in order to optimize adsorbent performance.

From the cumulative pore volume for BGHHM carbon, it can be seen that 90% of the pore volume is less than 30 nm, making the adsorbent highly micro-porous. The surface area for adsorption is about 1350 $m^2/g$. For comparison, the CG71M acrylic ester resin has an average pore diameter of 25 nm and a surface area of 500 $m^2/g$, according to the manufacturer. Further characterization of all the promising adsorbents should be performed, to confirm that micro-porous adsorbents with a majority of the non-polar pores less than 30 nm are preferred adsorbents for separating the impurities from the iodixanol in polar solvents.

The invention is illustrated further by the following examples that are not to be construed as limiting the invention in scope to the specific procedures or products described in them.

## EXAMPLES

### EXAMPLE 1: Reaction conditions

Reagents for adsorbent studies: 2-Methoxyethanol (2-ME; Aldrich, HPLC grade, >=99.9%, Lot#03758HE) was used without further purification. De-ionized water was provided by a Millipore Synergy 185 system, giving 18 mega-ohm water. Iodixanol solids were from Lindesnes Batch #10429846. Adsorbents used and their manufacturers are listed in Table 1.

**Table 1 Adsorbent types and manufacturers used for iodixanol purification**

| Adsorbent | Manufacturer |
|---|---|
| **Activated Carbons** | |
| GC 8x30S | General Carbon |
| Carbsorb 40 | Calgon |
| CPG | Calgon |
| OLC | Calgon |
| BL | Calgon |
| BG-HHM | Calgon |
| NC01-125 | Norit |
| M-1311 | Norit |
| **Polymers** | |
| CG161M Acrylic Ester | Rohm & Haas |
| CG71M Acrylic Ester | Rohm & Haas |
| XAD-7 Acrylic Ester | Rohm & Haas |
| XAD-4 Di-Vinyl Benzene | Rohm & Haas |
| XSD-2 Di-Vinyl Benzene | Rohm & Haas |

(continued)

| Silicas | |
|---|---|
| C930 | PQ Corporation |
| D350EL | PQ Corporation |

In addition, some other adsorbents were identified using the Supelco catalog, as potentially effective for these types of separations, and are listed in Table 2.

**Table 2 Listing of various polar and non-polar adsorbents available from Supelco**

| Adsorbent | Supelco# | Manufacturer | CAS# | Composition | Surface A Pore ($m^2/g$) | Pore V (ml/g) | Mean pore size (nm) | Particle Size |
|---|---|---|---|---|---|---|---|---|
| Sipeite DAX-8 | 20278 | Supelco | | Acrylic Ester | 160 | 0.79 | 22.5 | 40-60 Mesh |
| Combigel (A-nberlite) XE-305 | 502537B | Supelco, R&H | | Underivatized polystyrene | | | | 50-100 Mesh |
| P4EDVB | 42596-2 | Supelco | 9043-77-0 | Poly(4-ethylsyrene-co-DVB) | | | 10.0 | 300-1200 μm |
| P4EDVB | 42596-2 | Supelco | 9043-77-0 | Poly(4-ethylsyrene-co-DVB) | | | 7.5 | 500 μm |
| PS-DVB | 42698-9 | Supelco | 9003-70-7 | Poly(styene-co-DVB) | | | 4.6 | 300-800 μm |
| Amberlite XAD7HP | 13361-U | R&H | 37380-43-1 | Acrylic Ester | 450 | 1.14 | 9.0 | 20-00 Mesh |
| Amberlite XAD16HP | 133555-U | R&H | 9003-69-4 | PS-DVB | 800 | 1.82 | 10.0 | 20-00 Mesh |
| Amberlite XAD1180 | 10377 | R&H | 97396-56-0 | | 500 | | | |
| Amberlite XAD761 | 10356 | R&H | | phenolformaldehyde/ methylol FG | 300 | 0.43 | 60.0 | 16-50 Mesh |
| Amberchrom CG761 | 10366 | R&H | | Acrylic ester | 500 | 1.17 | 25.0 | 80-160 μm |
| Amberchrom CG161 | 10369 | R&H | | PS-DVB | 900 | 1.45 | 15.0 | 50-100 μm |
| Amberchrom CG300 | 13908 | R&H | | PS-DVB | 700 | 1.66 | 30.0 | 25-50 μm |
| Amberchrom CG1000S | 13911 | R&H | | PS-DVB | 250 | 1.66 | 100.0 | 25-50 μm |
| Diaion HP-20 | 13605 | Mitsubishi | | PS-DVB | 500 | 1.3 | 26.0 | 250-850 μm |
| Diaion HP-20SS | 13613-U | Mitsubishi | | PS-DVB | 500 | 1.3 | 26.0 | 75-150 μm |
| Diaion HP-2MG | 13601 | Mitsubishi | | PMA | 500 | 1.2 | 17.0 | 25-50 μm |

(continued)

| Adsorbent | Supelco# | Manufacturer | CAS# | Composition | Surface A Pore ($m^2/g$) | Pore V (ml/g) | Mean pore size (nm) | Particle Size |
|---|---|---|---|---|---|---|---|---|
| Sepabesds SP-20SS | 13617-U | Mitsubishi | | PS-DVB | 500 | 1.01 | 26.0 | 50-100 μm |

Adsorption Experimental: Adsorption studies used a standard solution of iodixanol in 2-ME or de-ionized water. Several controls of each solution were measured for iodixanol, backpeak and N-acetyl cyclics via HPLC. HPLC samples of solutions used in controls and adsorption experiments were prepared from a weighed 125-μl aliquot diluted in a weighed amount of 25 ml de-ionized water.

Silica and carbon adsorbents were prepared by drying at 250°C and 200 mm Hg vacuum in an oven in order to remove any adsorbed species prior to mixing with the solution. Polymer adsorbents were dried at 100°C and 200 mm Hg.

The adsorption was performed by weighing out 10 ml of standard solution into a 50-ml jar. Next a weighed amount of adsorbent was added to the jar, along with a Teflon stir bar. The jar was then sealed under nitrogen and mixed on a stir plate for 24-hours. The temperature of the ambient air was also monitored, and fluctuated between 22-27°C.

After equilibration, a portion of the resulting solution was filtered using a 0.5 μm Teflon syringe filter to remove any adsorbent particles. Then a 125-μl aliquot of this filtered solution was diluted in a weighed amount of 25 ml de-ionized water. This diluted sample was then analyzed via HPLC.

Subsequently, the areas for peaks of the known standard solutions were used to calculate the amount of iodixanol, backpeak and N-acetyl cyclics in the equilibrated adsorption solution. The dilution factor was taken into account, and the amounts of each component in the solution and adsorbed phases were calculated.

<u>HPLC Analysis of Adsorbent Solutions:</u> HPLC analysis was performed on an Agilent 1100 series HPLC as indicated below.

Column: YMC-Pack ODS-AM, S-5 μm, 120A, 15 cm and diameter 4.6 mm,

Mobile phase: - Millipore Synergy 185 system de-ionized water.

- 50 % Acetonitrile in Millipore Synergy 185 system de-ionized water.

| | |
|---|---|
| Flow: | 1.25 ml/min. |
| Detector: | UV - 254 nm (15 μl flow cell and 10 mm path length) |
| Temperature: | 25°C or ambient |
| Gradient: | |

| Time (min) | 50% ACN in de-ionized water | De-ionized water |
|---|---|---|
| 0.0 | 6 | 94 |
| 2.7 | 6 | 94 |
| 5.5 | 14 | 86 |
| 16.5 | 14 | 86 |
| 19.5 | 26 | 74 |
| 26.5 | 90 | 10 |
| 31.5 | 90 | 10 |

**EXAMPLE 2: Silica adsorption using 2-methoxyethanol solution**

157.5g of iodixanol (GE Healthcare Lot#1042986) was dissolved in 771.7 g of 2-Methoxyethanol (Aldrich Lot#03758HE) to give a 16.95 Wt% solids (iodixanol and impurities) in solution. 25 ml aliquots of this solution were added to a glass jar with a magnetic stir bar. All adsorbents were dried at 250°C and 250 mm Hg vacuum in a drying oven. Then a weighed amount of dry adsorbent was also added to the jars, except for the control, where no adsorbent was added. The amounts are summarized in Table 3, to give various iodixanol to adsorbent ratios. The glass jars were then placed on a magnetic stirring board and allowed to stir and equilibrate for 20 hours. The subsequent mother liquor was then filtered using a 2-micron Teflon syringe filter. A 125-μl aliquot of this filtered solution was then diluted in 25 ml of 16 mega-ohm purified water and analyzed for the amount of iodixanol, backpeaks and N-acetyl cyclized solids remaining in the solution. The relative amount of each component in each solution was then compared to the control to determine how effective the adsorbent was at removing the impurities. The data in Table 3 shows that the silica adsorbents are not effective at separating out the non-polar impurities from the iodixanol in 2-Methoxyethanol.

**Table 3 Summary of Silica Adsorption Experiments using Iodixanol dissolved in 2-Methoxyethanol**

| Notebool# # | AS326-78-1 | AS326-78-2 | AS326-78-3 | AS326-784 | A5326-78-8 | AS326-78-9 | AS326-78-10 | AS326-78-11 | AS326-78-12 | A5326-78-13 |
|---|---|---|---|---|---|---|---|---|---|---|
| Solution Mass (q) | 26.43 | 26.48 | 26.80 | 26.47 | 26.51 | 26.48 | 26.40 | 26.47 | 26.42 | 26.45 |
| Iodixand Content (g) | 4.4799 | 4.4884 | 4.5426 | 4.4867 | 4.4934 | 4.4884 | 4.4850 | 4.4867 | 4.4782 | 4.4833 |
| Adsorbent Type | None | D350EL Silica | D350EL Silica | D350EL Silica | R100 Silica | R100 Silica | R100 Silica | C930 Silica | C930 Slica | C930 Silica |
| Adsorbent Mass (g) | - | 0.44 | 0.91 | 2.24 | 2.25 | 0.89 | 0.44 | 0.44 | 0.89 | 2.24 |
| Manufacture | - | PQCorp. | PQCorp. | PQCorp. | PQCorp. | PQCorp. | PQCorp. | PQCorp. | PQCorp. | PQCorp. |
| Lot# | - | S009 | S009 | S009 | R40507313 | R40507313 | R40507313 | 52642 | 52642 | 52642 |
| Iodixand Mass/Solids Mass | - | 1020 | 4.99 | 2.00 | 2.00 | 5.04 | 10.19 | 1020 | 5.03 | 2.00 |
| Drying T (C) | - | 250 | 250 | 250 | 250 | 250 | 250 | 250 | 250 | 250 |
| | | | | | | | | | | |
| Equilibrium Analysis (Normalized % component in solution) | | | | | | | | | | |
| Iodixanol | 99.1918 | 99.0714 | 99.1934 | 99.0780 | 99.2387 | 98.9785 | 99.0692 | 98.9975 | 99.0964 | 99.1139 |
| Backpeaks | 0.7548 | 0.8144 | 0.7349 | 0.8084 | 0.7060 | 0.9206 | 0.8258 | 0.8959 | 0.8168 | 0.8106 |
| N-acetyl-cyclized | 0.0535 | 0.1142 | 0.0717 | 0.1136 | 0.0552 | 0.1010 | 0.1050 | 0.1066 | 0.0869 | 0.0755 |

**Example 3: Alumina adsorption using 2-methoxyethanol solution**

Using the same control solution prepared in Example 2, and the same method of drying the adsorbents, a similar set of experiments was done using Alumina adsorbent. The amounts are summarized in Table 4, to give various iodixanol to adsorbent ratios. The glass jars were then placed on a magnetic stirring board and allowed to stir and equilibrate for 20 hours. The subsequent mother liquor was then filtered using a 2-micron Teflon syringe filter. A 125-μl aliquot of this filtered solution was then diluted in 25 ml of 16 mega-ohm purified water and analyzed for the amount of iodixanol, backpeaks and N-acetyl cyclized solids remaining in the solution. The relative amount of each component in each solution was then compared to the control to determine how effective the adsorbent was at removing the impurities. The data in Table 4 show that the alumina adsorbents are not effective at separating out the non-polar impurities from the iodixanol in 2-Methoxyethanol, and in fact due to the acidic nature of the adsorbent surface, they catalyze the reaction of iodixanol to the N-acetyl cyclized impurity. The higher the amount of adsorbent added, the larger the N-acetyl cyclized impurity formed.

**Table 4 Summary of Alumina Adsorption Experiments using iodixanol dissolved in 2-Methoxyethanol**

| Notebook # | AS326-78-1 | AS326-78-5 | AS326-78-6 | AS326-78-7 |
|---|---|---|---|---|
| Solution Mass (g) | 26.43 | 26.50 | 26.44 | 26.50 |
| Iodixanol Content (g) | 4.4799 | 4.4918 | 4.4816 | 4.4918 |
| Adsorbent Type | None | Alumina 9139A | Alumina 9139A | Alumina 9139A |
| Adsorbent Mass (g) | - | 0.46 | 0.89 | 2.25 |
| Manufacturer | - | UOP | UOP | UOP |
| Lot# | - | 4413000226 | 4413000226 | 4413000226 |
| Iodixanol Mass/Solids Mass | - | 9.76 | 5.04 | 2.00 |
| Drying T (C) | - | 250 | 250 | 250 |
| | | | | |
| Equilibrium Analysis (Normalized % component in solution) | | | | |
| Iodixanol | 99.1918 | 98.1269 | 97.5749 | 96.6652 |
| Backpeaks | 0.7548 | 0.7008 | 0.7425 | 0.6906 |
| N-acetyl-cyclized | 0.0535 | 1.1723 | 1.6825 | 2.6443 |

**Example 4: Polymer resin adsorption using 2-methoxyethanol solution**

Using the same control solution prepared in Example 2, and the same method of drying the adsorbents at 100°C and 250 mm Hg vacuum, a similar set of experiments was done using polymeric resin adsorbents. The amounts are summarized in Table 5, to give various iodixanol to adsorbent ratios. The glass jars were then placed on a magnetic stirring board and allowed to stir and equilibrate for 20 hours. The subsequent mother liquor was then filtered using a 2-micron Teflon syringe filter. A 125-μl aliquot of this filtered solution was then diluted in 25 ml of 16 mega-ohm purified water and analyzed for the amount of iodixanol, backpeaks and N-acetyl cyclized solids remaining in the solution. The relative amount of each component in each solution was then compared to the control to determine how effective the adsorbent was at removing the impurities. The data in Table 5 shows that the polymer adsorbents are not effective at separating out the non-polar impurities from the iodixanol in 2-Methoxyethanol. Again, due to the acidic nature of the adsorbent surface, some polymer resins catalyze the reaction of iodixanol to the N-acetyl cyclized impurity.

**Table 5 Summary of Resin Adsorption Experiments Using Iodixanol Dissolved in 2-Methoxyethanol**

| Notebook # | AS326-78-1 | AS326-79-18 | AS326-79-19 | AS326-79-20 | AS326-79-21 | AS326-79-22 | AS326-79-23 | AS326-79-24 |
|---|---|---|---|---|---|---|---|---|
| Solution Mass (g) | 26.43 | 26.54 | 26.47 | 26.43 | 26.36 | 26.40 | 26.45 | 26.50 |
| Iodixanol Content (g) | 4.4799 | 4.4980 | 4.4862 | 4.4804 | 4.4675 | 4.4751 | 4.4824 | 4.4923 |
| Adsorbent Type | None | Amberlite XAD-2 | Amberlite XAD-2 | Amberlite XAD-4 | Amberlite XAD-7 | Dowex MSA | IRC76 | Amberlyst 15 |
| Adsorbent Mass (g) | - | 2.005 | 0.408 | 2.003 | 2.002 | 2.003 | 2.003 | 2.003 |
| Manufacturer | - | Rohm & Haas | Rohm & Haas | Rohm & Haas | Rohm & Haas | Dow | Rohm & Haas | Rohm & Haas |
| Lot# | - | 04917TZ | 04917TZ | 03803HF | 03729DF | 13107EA | 6.22E+06 | 210126 |
| Iodixanol Mass/ Solids Mass | - | 2.24 | 11.00 | 2.24 | 2.23 | 2.23 | 2.24 | 2.24 |
| Drying T (C) | - | 100 | 100 | 100 | 100 | 100 | 100 | 100 |
| | | | | | | | | |
| Equilibrium Analysis (Normalized % component in solution) | | | | | | | | |
| Iodixanol | 99.1918 | 98.2166 | 98.9811 | 98.5871 | 99.2723 | 99.1813 | 99.1963 | 99.0813 |
| Backpeaks | 0.7548 | 0.7480 | 0.7729 | 0.7597 | 0.6807 | 0.7596 | 0.7389 | 0.8711 |
| N-acetylcyclized | 0.0535 | 1.0354 | 0.2460 | 0.6532 | 0.0470 | 0.0590 | 0.0648 | 0.0476 |

**Example 5: Further Polymer Resin Adsorption using 2-Methoxyethanol Solution**

Using the same control solution prepared in Example 2, and the same method of drying the adsorbents at 40°C and 250 mm Hg vacuum, a similar set of experiments to Example 4 was done using new set of non-polar polymeric resin adsorbents. The amounts are summarized in Table 6, to give various iodixanol to adsorbent ratios. The glass jars were then placed on a magnetic stirring board and allowed to stir and equilibrate for 20 hours. The subsequent mother liquor was then filtered using a 2-micron Teflon syringe filter. A 125-μl aliquot of this filtered solution was then diluted in 25 ml of 16 mega-ohm purified water and analyzed for the amount of iodixanol, backpeaks and N-acetyl cyclized solids remaining in the solution. The relative amount of each component in each solution was then compared to the control to determine how effective the adsorbent was at removing the impurities. The data in Table 6 shows that the polymer adsorbents are not effective at separating out the non-polar impurities from the iodixanol in 2-Methoxyethanol.

**Table 6 Summary of Resin Adsorption Experiments using Iodixanol dissolved in 2-Methoxyethanol**

| Notebook # | AS326-99-1 | AS326-99-2 | AS326-99-3 | AS326-99-4 | AS326-99-5 | AS326-99-6 |
|---|---|---|---|---|---|---|
| Solution Mass (g) | 6.9212 | 6.7906 | 7.1947 | 7.1462 | 7.1619 | 7.1891 |
| Iodixanol Content (g) | 1.1731 | 1.1510 | 1.2195 | 1.2113 | 1.2139 | 1.2186 |
| Adsorbent Type | None | CG71M | CG71M | None | CG161M | CG161M |
| Adsorbent Mass (g) | - | 0.2302 | 0.6114 | - | 0.2428 | 0.6085 |
| Manufacturer | - | Rohm & Haas | Rohm & Haas | - | Rohm & Haas | Rohm & Haas |
| Lot# | - | 0003604478 | 0003604478 | - | 0003558506 | 0003558506 |
| Iodixanol Mass/Solids Mass | - | 5.000 | 1.995 | - | 5.000 | 2.003 |
| Drying T (C) | - | 100 | 100 | - | 100 | 100 |
| | | | | | | |
| Equilibrium Analysis (Normalized % component in solution) | | | | | | |
| Iodixanol | 99.1302 | 99.0317 | 99.0926 | 99.1604 | 99.2252 | 99.2095 |
| Backpeaks | 0.7859 | 0.8773 | 0.8397 | 0.7760 | 0.7153 | 0.7471 |
| N-acetyl-cydized | 0.0838 | 0.0910 | 0.0677 | 0.0636 | 0.0595 | 0.0435 |

**Example 6: Carbon Adsorption using Aqueous Solution**

55.05g of iodixanol (GE Healthcare Lot#1042986) was dissolved in 220.1 g of 16 mega-ohm purified water to give a 20.76 Wt% solids (iodixanol and impurities) in solution. 10 ml aliquots of this solution were added to a glass jar with a magnetic stir bar. All adsorbents were dried at 250°C and 250 mm Hg vacuum in a drying oven. Then a weighed amount of dry adsorbent was also added to the jars, except for the control, where no adsorbent was added. The amounts are summarized in Table 7, to give various iodixanol to adsorbent ratios. The glass jars were then placed on a magnetic stirring board and allowed to stir and equilibrate for 20 hours. The subsequent mother liquor was then filtered using a 2-micron Teflon syringe filter. A 125-μl aliquot of this filtered solution was then diluted in 25 ml of 16 mega-ohm purified water and analyzed for the amount of iodixanol, backpeaks and N-acetyl cyclized solids remaining in the solution. The relative amount of each component in each solution was then compared to the control to determine how effective the adsorbent was at removing the impurities. The data in Table 7 shows that some carbon adsorbents with the right

properties are effective at separating out the non-polar impurities from the iodixanol in a polar water solution.

**Table 7 Summary of Carbon Adsorption Experiments using Iodixanol dissolved in water**

| Notebook# | AS99-7 | AS99-8 | AS99-9 | AS99-10 | AS99-11 | AS99-12 | AS99-13 | AS99-14 | AS99-15 |
|---|---|---|---|---|---|---|---|---|---|
| Sdution Mass (g) | 10.9606 | 10.9628 | 10.9130 | 11.0316 | 10.9681 | 11.0744 | 11.0208 | 10.9792 | 11.0233 |
| Iodixanol Content (g) | 2.2754 | 2.2759 | 2.2655 | 2.2902 | 2.2770 | 2.2990 | 2.2879 | 2.2793 | 2.2884 |
| Adsorbent Type | None | Carbon 8x30S | Carbsorb 40 | CPG Carbon | OLC Carbon | BL Carbon | BG-HHM Carbon | NC01-125 Carbon | M-1311 Carbon |
| Adsorbent Mass (g) | - | 1.1387 | 1.1329 | 1.1452 | 1.1383 | 1.1518 | 1.1445 | 1.1402 | 1.1449 |
| Manufacturer | - | General Carbon | Calgon | Calgon | Calagon | Calgon | Calgon | Norit | Norit |
| Lot# | - | None-research | None-research | None-research | None - research | None - research | None-research | None-research | None-research |
| Iodixanol Masa/Solids Mass | - | 1.9989 | 1.9997 | 1.9999 | 2.0004 | 1.9960 | 1.9990 | 1.9990 | 1.9988 |
| Drying T (C) | - | 250 | 250 | 250 | 250 | 250 | 250 | 250 | 250 |
| | | | | | | | | | |
| Equilibrium Analysis (Normalized % component in solution) | | | | | | | | | |
| Iodixanol | 99.2216 | 99.2257 | 99.3391 | 99.3555 | 99.2463 | 99.4259 | 99.5267 | 99.5174 | 99.5022 |
| Backpeaks | 0.6662 | 0.7398 | 0.5853 | 0.6054 | 0.6835 | 0.5547 | 0.4382 | 0.4479 | 0.4704 |
| N-acetyl-cyclized | 0.1122 | 0.0345 | 0.0756 | 0.0391 | 0.0702 | 0.0194 | 0.0351 | 0.0347 | 0.0275 |

**Example 7: Polymer Resin and Polar Silica Adsorption using Aqueous Solution**

Using the same control solution prepared in Example 6, and the same method of drying the adsorbents at 100°C for polymer (40°C for CG series polymers) and 250°C for silica and 250 mm Hg vacuum, a similar set of experiments to Example 6 was done using new set of non-polar polymeric resin adsorbents and a set of polar silica for contrast. The amounts are summarized in Table 8, to give various iodixanol to adsorbent ratios. The glass jars were then placed on a magnetic stirring board and allowed to stir and equilibrate for 20 hours. The subsequent mother liquor was then filtered using a 2-micron Teflon syringe filter. A 125-μl aliquot of this filtered solution was then diluted in 25 ml of 16 mega-ohm purified water and analyzed for the amount of iodixanol, backpeaks and N-acetyl cyclized solids remaining in the solution. The relative amount of each component in each solution was then compared to the control to determine how effective the adsorbent was at removing the impurities. The data in Table 8 shows that some polymer adsorbents are effective at separating out the non-polar impurities from the iodixanol in water, while the polar silicas are not effective at iodixanol purification in a polar solvent like water.

**Table 8 Summary of Resin & Silica Adsorption Experiments using Iodixanol dissolved in water**

| Notebook # | AS99-23 | AS99-16 | AS99-17 | AS99-18 | AS99-19 | AS99-20 | AS99-21 | AS99-22 |
|---|---|---|---|---|---|---|---|---|
| Solution Mass (g) | 11.1108 | 10.9882 | 10.9293 | 11.0162 | 10.9395 | 10.9812 | 10.8965 | 10.9858 |
| Iodixanol Cortert (g) | 2.3066 | 2.2812 | 2.2689 | 2.2870 | 2.2710 | 2.2797 | 2.2621 | 2.2807 |
| Adsorbent Type | None | CG161M | CG71M | XAD-7 | C930 Silica | D350EL Silica | XAD-2 | XAD-4 |
| Adsorbent Mass (g) | - | 1.1438 | 1.1345 | 1.1511 | 1.1385 | 1.1422 | 1.1311 | 1.1402 |
| Manufacturer | - | Rohm & Haas | Rohm & Haas | Rohm & Haas | PQ Corp. | PQ Corp. | Rohm & Haas | Rohm & Haas |
| Lot# | - | 3558506 | 3604478 | 03729DF | 52642 | S009 | 04917TZ | 03803HF |
| Iodixanol Mass/Solids Mass | - | 1.9944 | 2.0000 | 1.9868 | 1.9947 | 1.9959 | 2.0000 | 2.0003 |
| Drying T (C) | - | 40 | 40 | 100 | 250 | 250 | 100 | 100 |
| | | | | | | | | |
| Equilibrium Analysis (Normalized % component in solution) | | | | | | | | |
| Iodixanol | 99.2391 | 99.301 | 99.558 | 99.431 | 99.2569 | 99.3377 | 99.2592 | 99.214 |
| Backpeaks | 0.6829 | 0.665 | 0.4197 | 0.5205 | 0.6938 | 0.627 | 0.6535 | 0.7246 |
| N-acetyl-cyclized | 0.078 | 0.034 | 0.0222 | 0.0484 | 0.0493 | 0.0352 | 0.0873 | 0.0614 |

All patents, journal articles, publications and other documents discussed and/or cited above are hereby incorporated by reference.

## Claims

1. A process for purifying iodixanol from N-acetyl cyclized iodixanol, cyclized iodixanol, or primary o-alkyl trimer using non-polar organic adsorbents, where the average pore diameter is smaller than about 30 nm.

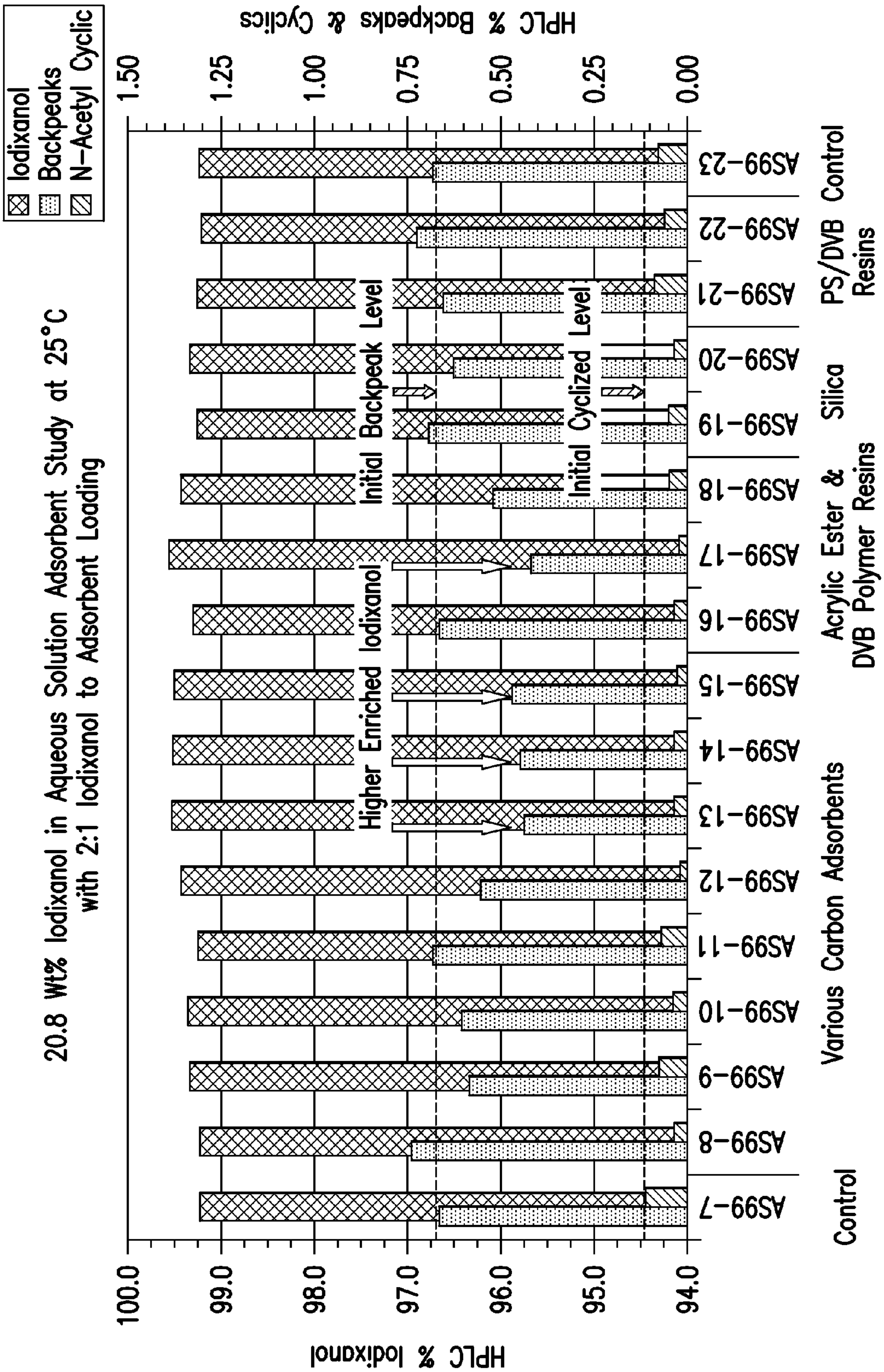
20.8 Wt% Iodixanol in Aqueous Solution Adsorbent Study at 25°C
with 2:1 Iodixanol to Adsorbent Loading
Iodixanol
Backpeaks
N-Acetyl Cyclic
HPLC % Backpeaks & Cyclics
1.50
1.25
1.00
0.75
0.50
0.25
0.00
HPLC % Iodixanol
100.0
99.0
98.0
97.0
96.0
95.0
94.0
Initial Backpeak Level
Initial Cyclized Level
Higher Enriched Iodixanol
AS99-7
AS99-8
AS99-9
AS99-10
AS99-11
AS99-12
AS99-13
AS99-14
AS99-15
AS99-16
AS99-17
AS99-18
AS99-19
AS99-20
AS99-21
AS99-22
AS99-23
Control
Various Carbon Adsorbents
Acrylic Ester & DVB Polymer Resins
Silica
PS/DVB Resins
Control

FIG.1

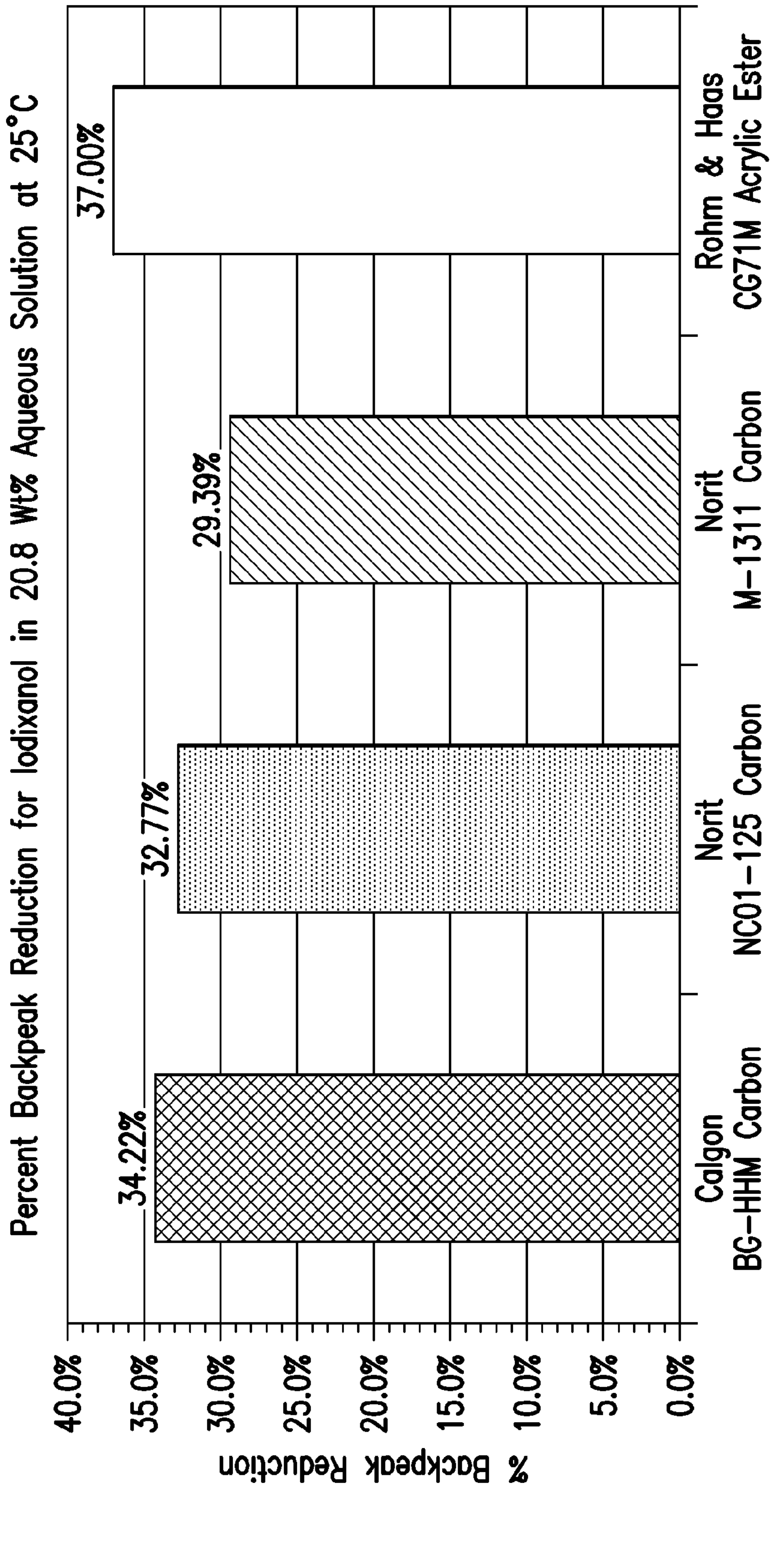
Percent Backpeak Reduction for Iodixanol in 20.8 Wt% Aqueous Solution at 25°C
34.22%
32.77%
29.39%
37.00%
Calgon
BG-HHM Carbon
Norit
NC01-125 Carbon
Norit
M-1311 Carbon
Rohm & Haas
CG71M Acrylic Ester
40.0%
35.0%
30.0%
25.0%
20.0%
15.0%
10.0%
5.0%
0.0%
% Backpeak Reduction

FIG. 2

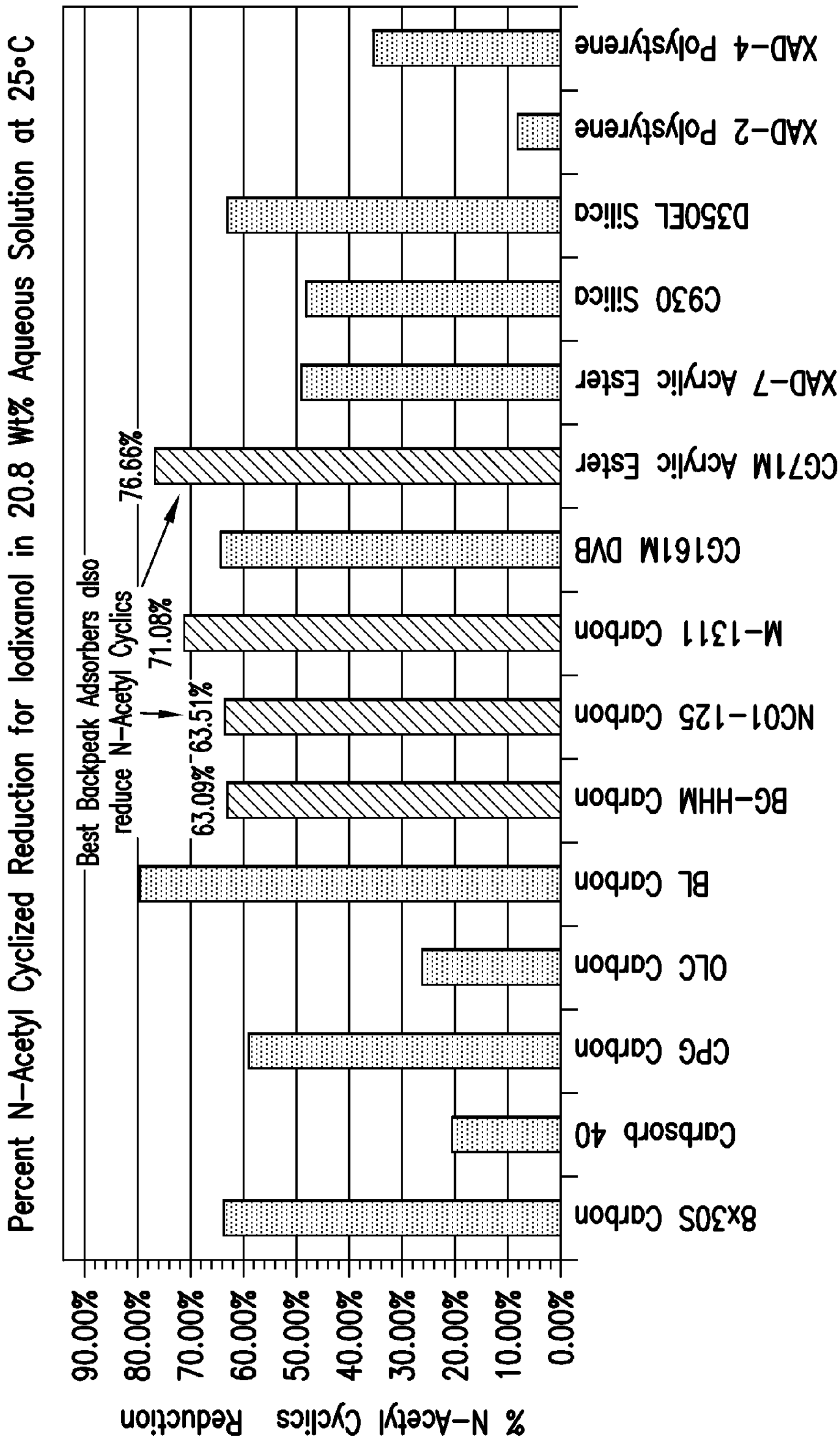
Percent N-Acetyl Cyclized Reduction for Iodixanol in 20.8 Wt% Aqueous Solution at 25°C
Best Backpeak Adsorbers also reduce N-Acetyl Cyclics
76.66%
71.08%
63.09%
63.51%
XAD-4 Polystyrene
XAD-2 Polystyrene
D350EL Silica
C930 Silica
XAD-7 Acrylic Ester
CG71M Acrylic Ester
CG161M DVB
M-1311 Carbon
NC01-125 Carbon
BG-HHM Carbon
BL Carbon
OLC Carbon
CPG Carbon
Carbsorb 40
8x30S Carbon
90.00%
80.00%
70.00%
60.00%
50.00%
40.00%
30.00%
20.00%
10.00%
0.00%
% N-Acetyl Cyclics Reduction

FIG.3

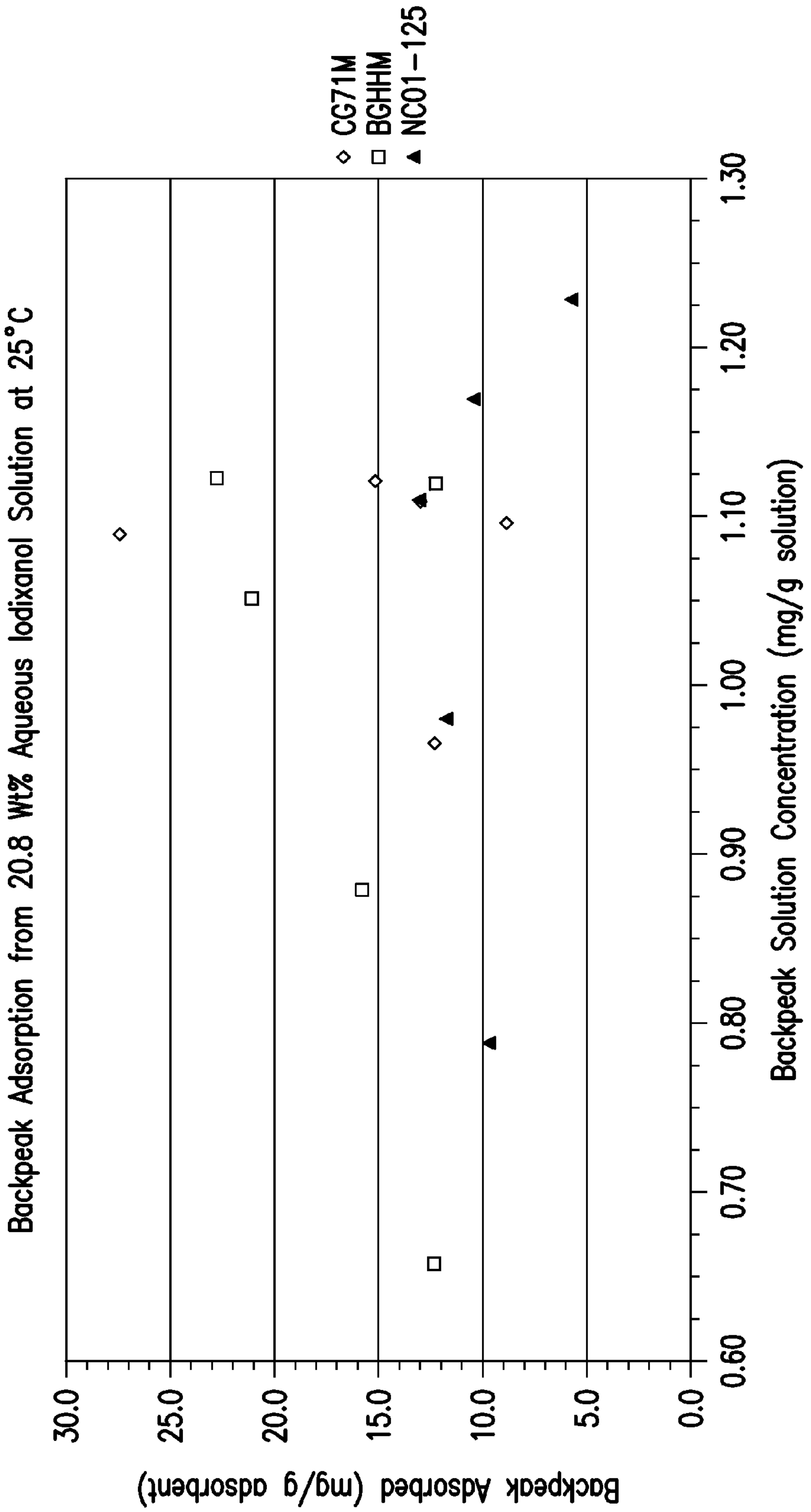
Backpeak Adsorption from 20.8 Wt% Aqueous Iodixanol Solution at 25°C
CG71M
BGHHM
NC01-125
Backpeak Adsorbed (mg/g adsorbent)
30.0
25.0
20.0
15.0
10.0
5.0
0.0
0.60
0.70
0.80
0.90
1.00
1.10
1.20
1.30
Backpeak Solution Concentration (mg/g solution)

FIG.4

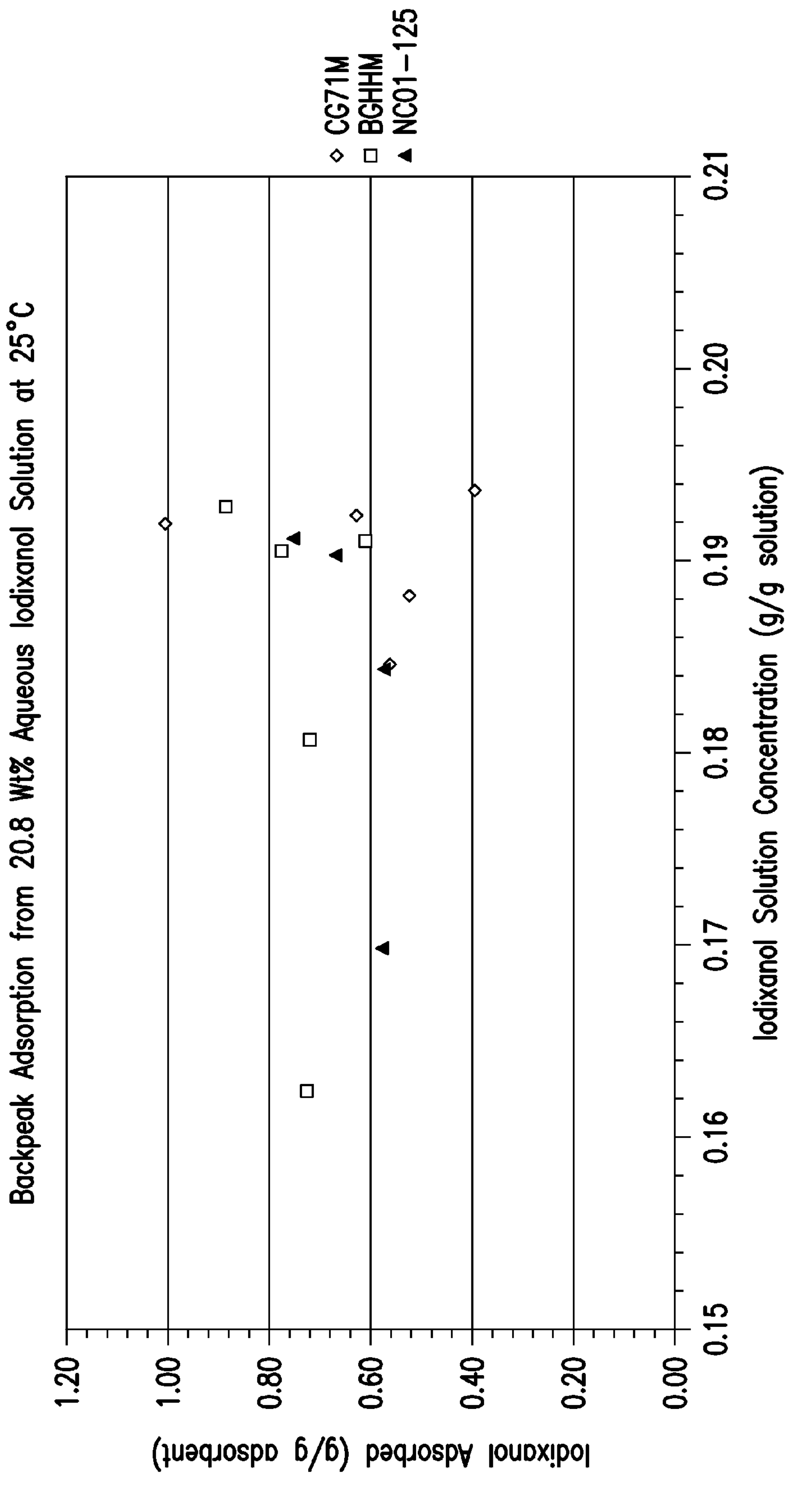
Backpeak Adsorption from 20.8 Wt% Aqueous Iodixanol Solution at 25°C
CG71M
BGHHM
NC01-125
1.20
1.00
0.80
0.60
0.40
0.20
0.00
Iodixanol Adsorbed (g/g adsorbent)
0.15
0.16
0.17
0.18
0.19
0.20
0.21
Iodixanol Solution Concentration (g/g solution)

FIG.5

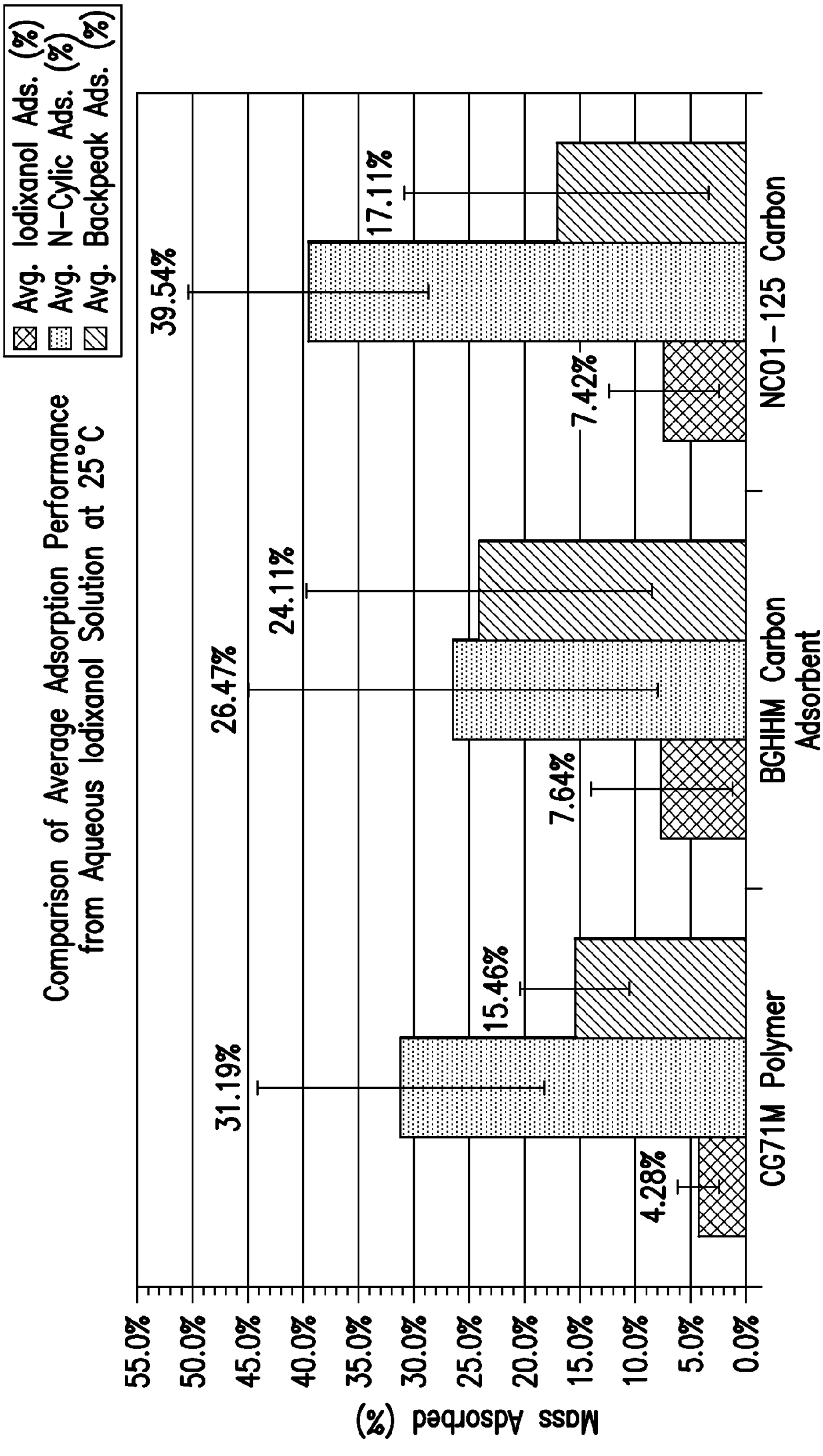
Comparison of Average Adsorption Performance
from Aqueous Iodixanol Solution at 25°C
Avg. Iodixanol Ads. (%)
Avg. N-Cylic Ads. (%)
Avg. Backpeak Ads. (%)
Mass Adsorbed (%)
55.0%
50.0%
45.0%
40.0%
35.0%
30.0%
25.0%
20.0%
15.0%
10.0%
5.0%
0.0%
4.28%
31.19%
15.46%
7.64%
26.47%
24.11%
7.42%
39.54%
17.11%
CG71M Polymer
BGHHM Carbon Adsorbent
NC01-125 Carbon

FIG.6

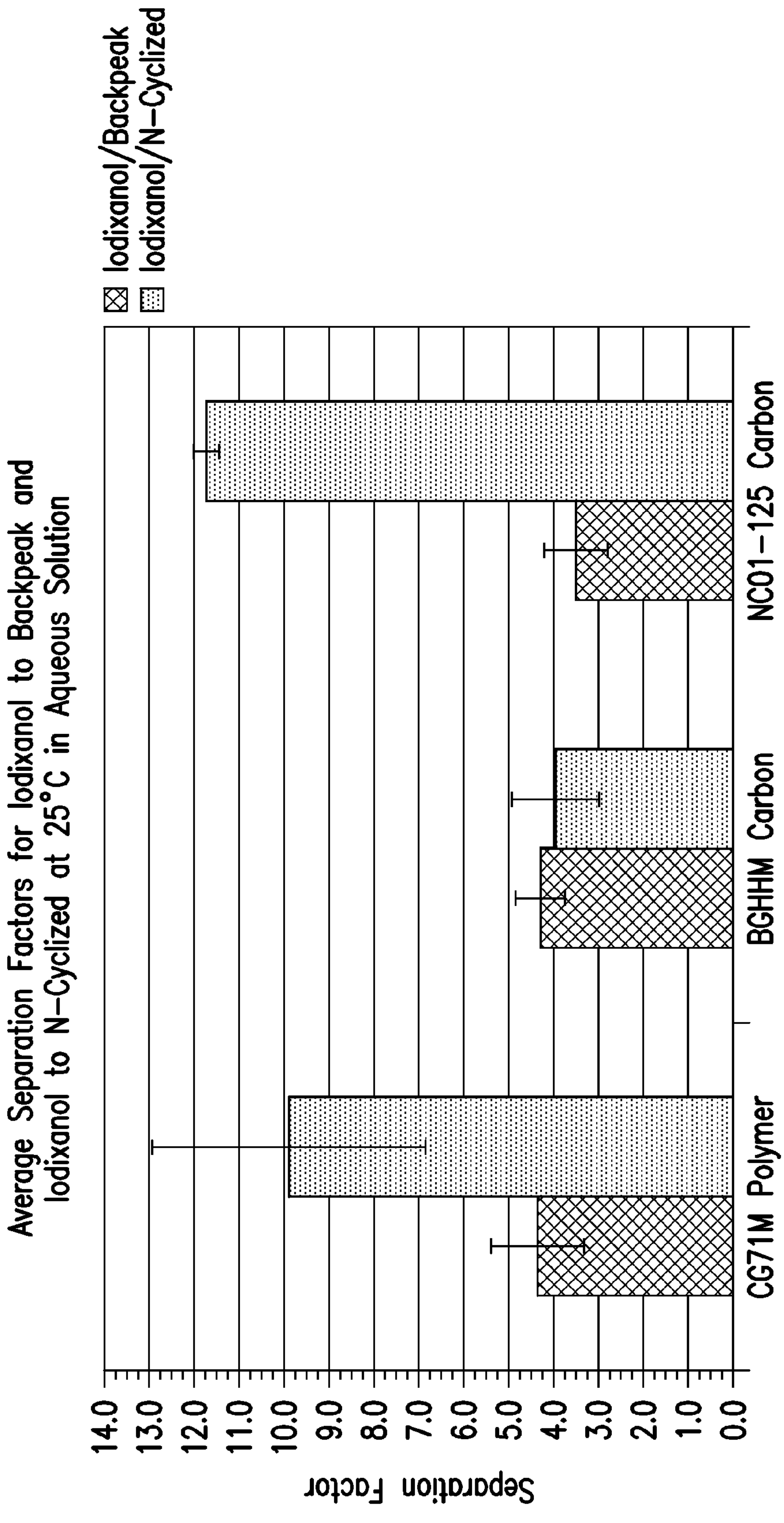
Average Separation Factors for Iodixanol to Backpeak and
Iodixanol to N-Cyclized at 25°C in Aqueous Solution
Iodixanol/Backpeak
Iodixanol/N-Cyclized
Separation Factor
14.0
13.0
12.0
11.0
10.0
9.0
8.0
7.0
6.0
5.0
4.0
3.0
2.0
1.0
0.0
CG71M Polymer
BGHHM Carbon
NC01-125 Carbon

FIG.7

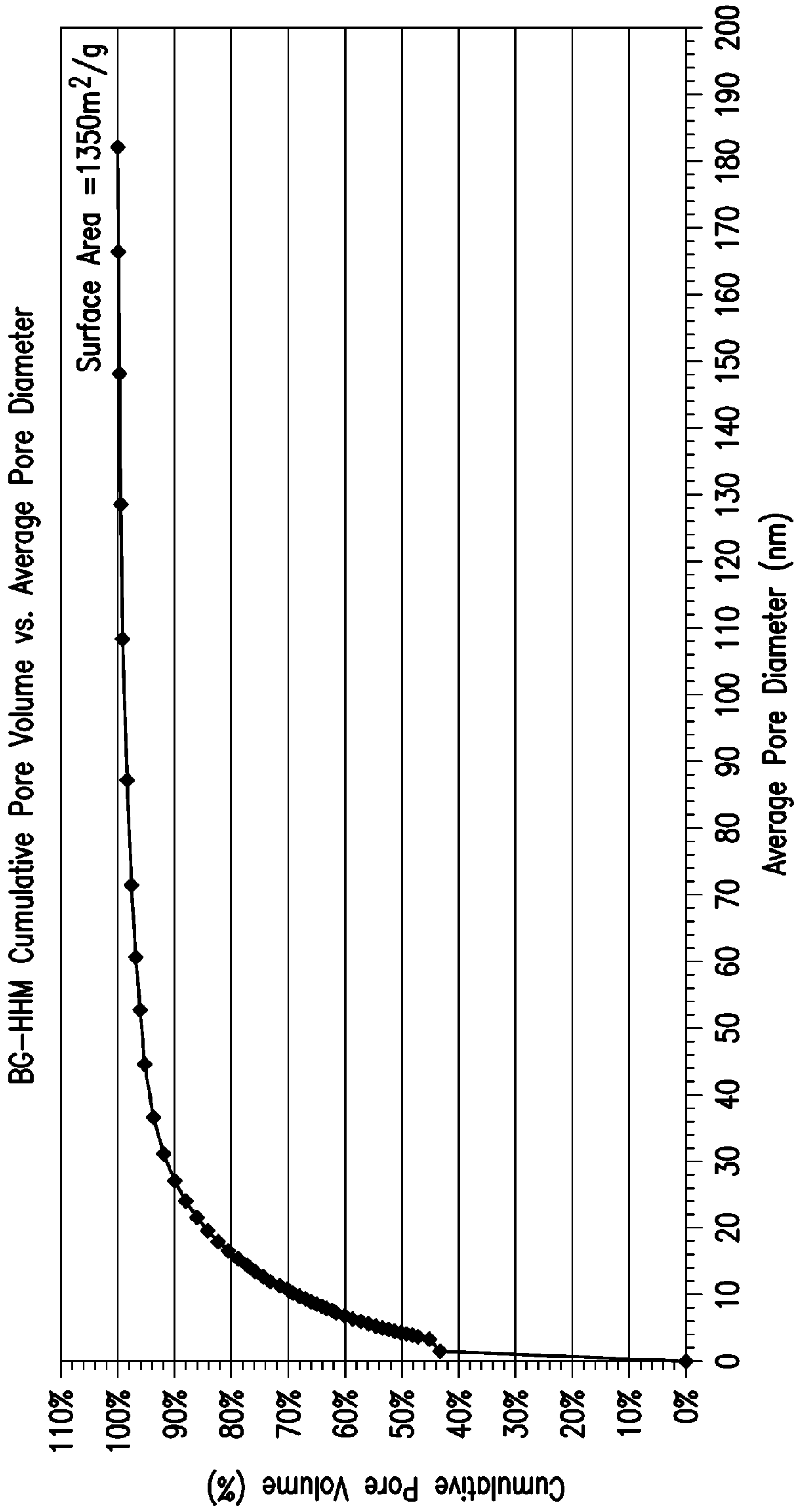
BG-HHM Cumulative Pore Volume vs. Average Pore Diameter
Surface Area =1350m²/g
Cumulative Pore Volume (%)
110%
100%
90%
80%
70%
60%
50%
40%
30%
20%
10%
0%
0
10
20
30
40
50
60
70
80
90
100
110
120
130
140
150
160
170
180
190
200
Average Pore Diameter (nm)

FIG.8

Europäisches Patentamt
European Patent Office
Office européen des brevets

# EUROPEAN SEARCH REPORT

Application Number
EP 09 17 6526

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 204 005 A (DORAN III NARCISO O [US] ET AL) 20 April 1993 (1993-04-20)<br>* column 4, line 18 - line 38 *<br>* column 4, line 48 - line 50 *<br>----- | 1 | INV.<br>C07C231/24<br>C07C237/46<br>C07B63/00<br>B01J20/281 |
| X | US 2005/192465 A1 (LORENZINI RICHARD A [US] ET AL) 1 September 2005 (2005-09-01)<br>[0029, 0031, 0047]* example 3 *<br>----- | 1 | |
| X | WO 2008/104853 A (WOCKHARDT RES CT [IN]; KULKARNI DILIP GANESH [IN]; SINHA ROHIT KUMAR [) 4 September 2008 (2008-09-04)<br>* page 10, line 5 - line 8; claim 13; examples 4,5 *<br>----- | 1 | |
| X | WO 92/08691 A (DIBRA SPA [IT]; BRACCO IND CHIMICA SPA [IT]) 29 May 1992 (1992-05-29)<br>* page 19; examples 2,19 *<br>----- | 1 | |
| A | WO 2007/073202 A (GE HEALTHCARE AS [NO]; HOMESTAD OLE MAGNE [NO])<br>28 June 2007 (2007-06-28)<br>* the whole document *<br>----- | 1 | TECHNICAL FIELDS SEARCHED (IPC)<br>C07C<br>B01D<br>C07B<br>B01J |
| A | WO 2007/064220 A (GE HEALTHCARE AS [NO]; STRANDMYR BJOERG HELGA MELLESD [NO])<br>7 June 2007 (2007-06-07)<br>* the whole document *<br>----- | 1 | |

The present search report has been drawn up for all claims

1

| Place of search | Date of completion of the search | Examiner |
|---|---|---|
| Munich | 9 February 2010 | Breimaier, Waltraud |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 09 17 6526

This annex lists the patent family members relating to the patent documents cited in the above-mentioned European search report. The members are as contained in the European Patent Office EDP file on
The European Patent Office is in no way liable for these particulars which are merely given for the purpose of information.

09-02-2010

| Patent document cited in search report | | Publication date | Patent family member(s) | | | Publication date |
|---|---|---|---|---|---|---|
| US 5204005 | A | 20-04-1993 | AT | 151655 | T | 15-05-1997 |
| | | | AU | 626755 | B2 | 06-08-1992 |
| | | | CA | 2062962 | A1 | 27-08-1991 |
| | | | DE | 69125665 | D1 | 22-05-1997 |
| | | | DE | 69125665 | T2 | 30-10-1997 |
| | | | DK | 0470247 | T3 | 27-10-1997 |
| | | | EP | 0470247 | A1 | 12-02-1992 |
| | | | ES | 2103309 | T3 | 16-09-1997 |
| | | | GR | 3023716 | T3 | 30-09-1997 |
| | | | IE | 910617 | A1 | 28-08-1991 |
| | | | NO | 920175 | A | 14-01-1992 |
| | | | WO | 9112868 | A1 | 05-09-1991 |
| US 2005192465 | A1 | 01-09-2005 | NONE | | | |
| WO 2008104853 | A | 04-09-2008 | NONE | | | |
| WO 9208691 | A | 29-05-1992 | AT | 111441 | T | 15-09-1994 |
| | | | AU | 646119 | B2 | 10-02-1994 |
| | | | CA | 2096136 | A1 | 17-05-1992 |
| | | | DE | 69104059 | D1 | 20-10-1994 |
| | | | DE | 69104059 | T2 | 02-02-1995 |
| | | | DE | 557345 | T1 | 28-07-1994 |
| | | | DK | 0557345 | T3 | 06-03-1995 |
| | | | EP | 0557345 | A1 | 01-09-1993 |
| | | | ES | 2060415 | T3 | 16-11-1994 |
| | | | GR | 93300134 | T1 | 28-02-1994 |
| | | | HU | 65652 | A2 | 28-07-1994 |
| | | | IE | 913986 | A1 | 20-05-1992 |
| | | | IL | 100054 | A | 14-05-1996 |
| | | | IS | 3781 | A | 17-05-1992 |
| | | | IT | 1245853 | B | 25-10-1994 |
| | | | JP | 2977613 | B2 | 15-11-1999 |
| | | | JP | 6504268 | T | 19-05-1994 |
| | | | MX | 9102103 | A1 | 01-06-1992 |
| | | | NZ | 240566 | A | 25-02-1994 |
| | | | ZA | 9109065 | A | 26-08-1992 |
| WO 2007073202 | A | 28-06-2007 | EP | 1966110 | A1 | 10-09-2008 |
| | | | US | 2008300423 | A1 | 04-12-2008 |
| WO 2007064220 | A | 07-06-2007 | EP | 1960349 | A1 | 27-08-2008 |
| | | | US | 2008287711 | A1 | 20-11-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **King, C.J.** Separation Processes. McGraw-Hill, 1980